# EUROPEAN PATENT APPLICATION

(11) **EP 4 667 896 A1**
(43) Date of publication of application: **24.12.2025**
(21) Application number: 23923851.2
(22) Date of filing: 13.12.2023
(51) Int. Cl.: G01M 99/00, A61B 18/12, G06F 30/27, G06N 3/0499, G06N 3/08

(54) **ELECTROPORATION ABLATION PERFORMANCE TEST APPARATUS AND METHOD UTILIZING HIGH-FREQUENCY BIDIRECTIONAL PULSES**

(30) Priority: 20.02.2023 CN 202310138563
(71) Applicant: Tianjin Intelligent Health Co., Ltd, Tianjin 300392 (CN)
(72) Inventor: ZHANG, Xiaochen, Tianjin 300392 (CN)
(74) Representative: Chung, Hoi Kan
(86) International application number: PCT/CN2023/138604
(87) International publication number: WO 2024/174688

(57) **Abstract**

The present invention provides an apparatus and a method for testing an electroporation ablation performance using high-frequency bidirectional pulses. The apparatus includes a pulse generation mechanism, which includes a bipolar pulse generator and a pulse control unit and is configured for generating high-frequency bidirectional pulses targeting a cell suspension of a cell assembly of interest; a traversal analysis device, which is configured for traversing respective numerical value combinations of a target pulse frequency, a set electric field intensity and a set length of time for the cell assembly of interest consisting of a fixed total number of living cells from a set part, so as to predict respective cell inhibition proportions and respective low-loss ablation performance levels corresponding to the respective numerical value combinations, respectively; and a prioritization analysis mechanism, which is configured for selecting, based on prediction data, a numerical value combination with an optimal ablation performance. According to the present invention, respective optimized ablation parameter combinations for structs to be ablated that are from different parts and have different volumes can be acquired in an artificial intelligent mode and a numerical value simulation-based traversal mode, while an electroporation ablation system with high efficiency and automated design is introduced.

## Description

### Technical Field

The present invention belongs to the field of electroporation by high-frequency bidirectional pulses, and in particular, relates to an apparatus and a method for testing an electroporation ablation performance using high-frequency bidirectional pulses.

### Description of Related Art

When ablation treatment is performed on cell assemblies from various parts, for example, the pig liver, high-frequency bipolar pulses at a micron frequency level show a better ablation effect as compared to high-frequency bipolar pulses at a millimeter frequency level. Specifically, despite the slightly less effective outcome in increasing the proportion of necrotic cells at the part of interest than the high-frequency bipolar pulses at the millimeter frequency level, the high-frequency bipolar pulses at the micron frequency level can acquire BAX, BCL-2, Caspase-3 and Caspase-9 apoptosis proteins by acting on mitochondria to collapse the mitochondrial membrane potential.

That is to say, the performance of the high-frequency bipolar pulses at the millimeter frequency level in terms of cell inhibition proportion can only lie in the specific value of the proportion of necrotic cells, but the performance of the high-frequency bipolar pulses at the micron frequency level in terms of cell inhibition proportion not only lies in the specific value of the proportion of necrotic cells, but also in the specific value of the proportion of apoptotic cells. That is, cells from the diseased part die off in manners of both necrosis and apoptosis after ablation treatment.

Exemplarily, a Chinese invention patent CN110946642 A in its disclosed text proposes a high-frequency bipolar irrecoverable electroporation system, which includes an upper information management module, a lower computer control module and a bipolar high-voltage pulse discharging circuit. The upper information management module receives set working parameters and transmits them to the lower computer control module, which then generates a control signal and transmits it to the bipolar high-voltage pulse discharging circuit to generate bipolar high-voltage pulses. Compared with the conventional unipolar irreversible electroporation pulse sequence, this invention can better and more uniformly increase the induced transmembrane potential of the close-packed cells to an analog electroporation threshold, thereby making an ablated region more uniform. This invention can achieve a better tumor ablation effect to achieve the purpose of inhibiting tumor growth, showing good safety and effectiveness.

Exemplarily, a Chinese invention patent CN106877729 A in its disclosed text proposes a high-frequency irreversible electroporator, which includes a power conversion circuit, an energy storage circuit, an isolated high-frequency conversion output circuit, a signal controller, an electrode and a power supply. The output of the power supply is connected to the power conversion circuit; the power conversion circuit is connected to the electrode via the energy storage circuit and the isolated high-frequency conversion output circuit in sequence; and the signal controller is connected to the control inputs of the energy storage circuit and the isolated high-frequency conversion output circuit, respectively. In this way, the pulse output signal of the isolated high-frequency conversion output circuit is controlled by the signal controller to allow for discharging of the electrode. This invention allows for irreversible electrical breakdown of living cells, increases the uniformity of irreversible electroporation, reduces the muscle contraction caused by electroporation-induced nerve simulation, and can be applied to the experimental studies on in vivo or ex vivo treatment of solid tumors.

However, there is still room for optimization in the performance and automation level of the technical solution of electroporation based on high-frequency bipolar pulses in the prior art. Also, for structs to be ablated that are from different parts and have different volumes, it is necessary to acquire their corresponding optimal ablation parameter combinations by carrying out complex numerical value combinations and cumbersome massive tests on many ablation parameters such as pulse frequency, electric field intensity and ablation length of time. Obviously, such an operation mode easily leads to cumbersome and lengthy test analysis processes.

### SUMMARY OF THE INVENTION

To solve the technical problems described above, the present invention proposes an apparatus for testing an electroporation ablation performance using high-frequency bidirectional pulses. On the basis of introduced hardware with high efficiency and automated design for an electroporation ablation system based on high-frequency bipolar pulses, different prediction models are customized for structs to be ablated that are from different parts and have different volumes, respective ablation result data are reliably predicted using massive respective ablation parameter combinations and the numerical value simulation analysis mode of for example the MATLAB toolbox, respectively, and then, respective optimized ablation parameter combinations for the structs to be ablated that are from different parts and have different volumes are acquired by means of a customized selection mechanism. In this way, low-loss ablation solutions with optimal effects are rapidly provided for the structs to be ablated that are from different parts and have different volumes, such that the damage to living structs can be reduced as much as possible, while the ablation effect is ensured.

Specifically, in a first aspect of the present invention, an apparatus for testing an electroporation ablation performance using high-frequency bidirectional pulses is proposed. The apparatus comprises:
a pulse generation mechanism, which comprises a bipolar pulse generator and a pulse control unit, wherein the pulse control unit is connected to the bipolar pulse generator and is configured for controlling a pulse generation mode of the bipolar pulse generator to generate high-frequency bidirectional pulses targeting a cell suspension of a cell assembly of interest;
a suspension preparation mechanism, which comprises a saline washing device, a digestive treatment device, a centrifuge, a mechanical arm, a timing device, a positioning device, a first preparation container, a second preparation container and a culture formulation device, wherein the suspension preparation mechanism is configured for preparing a cell suspension with a cell population density of 5 × 10⁵ mL⁻¹ by washing a cell assembly of interest two times with a phosphate buffer saline solution with a volume of 1 mL to 2 mL, then digesting the cell assembly of interest for 2 minutes with a pancreatin solution with a mass concentration of 0.5 g/L, then centrifuging with the centrifuge, discarding a supernatant, and adding a 1640 culture medium, and the cell assembly of interest is a struct consisting of a plurality of living cells from the same part;
an ablation treatment mechanism, which is configured for performing ablation treatment on the cell suspension with a set electric field intensity using high-frequency bidirectional pulses at a target pulse frequency, to obtain a treated solution after the ablation treatment has reached a set length of time;
a data measurement device, which is configured for measuring a total number of remaining living cells, a proportion of necrotic cells, and a proportion of apoptotic cells in the treated solution after the ablation treatment has reached a set length of time, and determining a cell inhibition proportion achieved by the ablation treatment and a low-loss ablation performance level, based on the proportion of necrotic cells and the proportion of apoptotic cells;
a network construction device, which is configured for establishing a deep feed-forward network to perform ablation performance prediction by taking the target pulse frequency, the set electric field intensity and the set length of time as used for performing the ablation treatment using the high-frequency bidirectional pulses as well as the total number of living cells in the cell assembly of interest as multiple input data of the deep feed-forward network, and by taking the cell inhibition proportion achieved by the ablation treatment and the low-loss ablation performance level as two output data of the deep feed-forward network;
a successive learning device, which is connected to the network construction device and is configured for performing a learning action on the deep feed-forward network by taking the target pulse frequency, the set electric field intensity and the set length of time as used for each ablation treatment as well as the total number of living cells in the cell assembly of interest as the multiple input data of the deep feed-forward network, and by taking the cell inhibition proportion achieved by each ablation treatment and the low-loss ablation performance level as two output data of the deep feed-forward network, and outputting the deep feed-forward network that has undergone multiple learning actions up to a preset total number, as an intelligent predictor;
a traversal analysis device, which is connected to the successive learning device and is configured for traversing respective numerical value combinations of the target pulse frequency, the set electric field intensity and the set length of time for the cell assembly of interest consisting of a fixed total number of living cells from a set part, so as to perform a prediction operation on the intelligent predictor of each numerical value combination and thus obtain respective cell inhibition proportions and respective low-loss ablation performance levels corresponding to the respective numerical value combinations, wherein the traversing is based on numerical value simulation treatment; and
a prioritization analysis mechanism, which is connected to the traversal analysis device and is configured for selecting, based on the respective cell inhibition proportions and the respective low-loss ablation performance levels corresponding to the respective numerical value combinations, a numerical value combination with an optimal ablation performance as an optimized ablation numerical value combination for the cell assembly of interest consisting of the fixed total number of living cells from the set part.

Specifically, in a second aspect of the present invention, a method for testing an electroporation ablation performance using high-frequency bidirectional pulses is proposed. The method comprises:
using a pulse generation mechanism, which comprises a bipolar pulse generator and a pulse control unit, wherein the pulse control unit is connected to the bipolar pulse generator and is configured for controlling a pulse generation mode of the bipolar pulse generator to generate high-frequency bidirectional pulses targeting a cell suspension of a cell assembly of interest;
using a suspension preparation mechanism, which comprises a saline washing device, a digestive treatment device, a centrifuge, a mechanical arm, a timing device, a positioning device, a first preparation container, a second preparation container and a culture formulation device, wherein the suspension preparation mechanism is configured for preparing a cell suspension with a cell population density of 5 × 10⁵ mL⁻¹ by washing a cell assembly of interest two times with a phosphate buffer saline solution with a volume of 1 mL to 2 mL, then digesting the cell assembly of interest for 2 minutes with a pancreatin solution with a mass concentration of 0.5 g/L, then centrifuging with the centrifuge, discarding a supernatant, and adding a 1640 culture medium, and the cell assembly of interest is a struct consisting of a plurality of living cells from the same part;
using an ablation treatment mechanism, which is configured for performing ablation treatment on the cell suspension with a set electric field intensity using high-frequency bidirectional pulses at a target pulse frequency, to obtain a treated solution after the ablation treatment has reached a set length of time;
using a data measurement device, which is configured for measuring a total number of remaining living cells, a proportion of necrotic cells, and a proportion of apoptotic cells in the treated solution after the ablation treatment has reached a set length of time, and determining a cell inhibition proportion achieved by the ablation treatment and a low-loss ablation performance level, based on the proportion of necrotic cells and the proportion of apoptotic cells;
using a network construction device, which is configured for establishing a deep feed-forward network to perform ablation performance prediction by taking the target pulse frequency, the set electric field intensity and the set length of time as used for performing the ablation treatment using the high-frequency bidirectional pulses as well as the total number of living cells in the cell assembly of interest as multiple input data of the deep feed-forward network, and by taking the cell inhibition proportion achieved by the ablation treatment and the low-loss ablation performance level as two output data of the deep feed-forward network;
using a successive learning device, which is connected to the network construction device and is configured for performing a learning action on the deep feed-forward network by taking the target pulse frequency, the set electric field intensity and the set length of time as used for each ablation treatment as well as the total number of living cells in the cell assembly of interest as the multiple input data of the deep feed-forward network, and by taking the cell inhibition proportion achieved by each ablation treatment and the low-loss ablation performance level as two output data of the deep feed-forward network, and outputting the deep feed-forward network that has undergone multiple learning actions up to a preset total number, as an intelligent predictor;
using a traversal analysis device, which is connected to the successive learning device and is configured for traversing respective numerical value combinations of the target pulse frequency, the set electric field intensity and the set length of time for the cell assembly of interest consisting of a fixed total number of living cells from a set part, so as to perform a prediction operation on the intelligent predictor of each numerical value combination and thus obtain respective cell inhibition proportions and respective low-loss ablation performance levels corresponding to the respective numerical value combinations, wherein the traversing is based on numerical value simulation treatment; and
using a prioritization analysis mechanism, which is connected to the traversal analysis device and is configured for selecting, based on the respective cell inhibition proportions and the respective low-loss ablation performance levels corresponding to the respective numerical value combinations, a numerical value combination with an optimal ablation performance as an optimized ablation numerical value combination for the cell assembly of interest consisting of the fixed total number of living cells from the set part.

Hence, it can be seen that the present invention has at least the following four key inventive points.
(1) An ablation system with a customized structure is used, which includes a pulse generation mechanism, a suspension preparation mechanism and an ablation treatment mechanism is used. The pulse generation mechanism includes a bipolar pulse generator and a pulse control unit. The suspension preparation mechanism includes a saline washing device, a digestive treatment device, a centrifuge, a mechanical arm, a timing device, a positioning device, a first preparation container, a second preparation container and a culture formulation device. In this way, targeted ablation treatment can be achieved for a plurality of structs to be ablated that consist of living cells from the same part, such that the performance and automation level of the electroporation ablation treatment using high-frequency bidirectional pulses are increased.
(2) A deep feed-forward network to perform ablation performance prediction is established by taking the target pulse frequency, the set electric field intensity and the set length of time as used for performing the ablation treatment using the high-frequency bidirectional pulses as well as the total number of living cells in the cell assembly of interest as multiple input data of the deep feed-forward network, and by taking the cell inhibition proportion achieved by the ablation treatment and the low-loss ablation performance level as two output data of the deep feed-forward network. A successive learning operation is performed on the deep feed-forward network to obtain an intelligent predictor capable of predicting an ablation performance.
(3) Respective numerical value combinations of the target pulse frequency, the set electric field intensity and the set length of time are traversed for a struct consisting of a fixed total number of living cells from a set part, so as to perform a prediction operation on the intelligent predictor of each numerical value combination and thus obtain respective cell inhibition proportions and respective low-loss ablation performance levels corresponding to the respective numerical value combinations. The traversing operation is based on numerical simulation treatment to thus achieve high-efficiency and reliable replacement for massive tests.
(4) Based on the respective cell inhibition proportions and the respective low-loss ablation performance levels corresponding to the respective numerical value combinations, the numerical value combination with the optimal ablation performance is selected as the optimized ablation numerical value combination for the struct consisting of the fixed total number of living cells from the set part. Moreover, a targeted prioritization mechanism is used, in which a numerical value combination with the highest low-loss ablation performance level at the preceding cell inhibition proportion and at an equivalent cell inhibition proportion is selected as the numerical value combination with the optimal ablation performance. In this way, the optimal ablation numerical value combination with the optimal ablation performance can be acquired for structs of different types and volumes, which further avoids cumbersome and lengthy test analysis processes.

### BRIEF DESCRIPTION OF THE SEVERAL VIEWS OF THE DRAWINGS

To describe the technical solutions in the embodiments of the present invention or in the prior art more clearly, the following briefly introduces the accompanying drawings required for describing the embodiments. Apparently, the accompanying drawings in the following description show merely some embodiments of the present invention, and a person of ordinary skill in the art may still derive other drawings from these accompanying drawings without creative efforts.
FIG. 1 shows a schematic diagram of inventive concepts of an apparatus and a method for testing an electroporation ablation performance using high-frequency bidirectional pulses according to the present invention;
FIG. 2 shows a schematic diagram of an internal structure of an apparatus for testing an electroporation ablation performance using high-frequency bidirectional pulses according to a first embodiment of the present invention;
FIG. 3 shows a schematic diagram of an internal structure of an apparatus for testing an electroporation ablation performance using high-frequency bidirectional pulses according to a second embodiment of the present invention;
FIG. 4 shows a schematic diagram of an internal structure of an apparatus for testing an electroporation ablation performance using high-frequency bidirectional pulses according to a third embodiment of the present invention;
FIG. 5 shows a schematic diagram of an internal structure of an apparatus for testing an electroporation ablation performance using high-frequency bidirectional pulses according to a fourth embodiment of the present invention;
FIG. 6 shows a schematic diagram of an internal structure of an apparatus for testing an electroporation ablation performance using high-frequency bidirectional pulses according to a fifth embodiment of the present invention; and
FIG. 7 shows a schematic diagram of a step process of a method for testing an electroporation ablation performance using high-frequency bidirectional pulses according to a sixth embodiment of the present invention.

### DETAILED DESCRIPTION OF THE INVENTION

The embodiments of the apparatus and method for testing an electroporation ablation performance using high-frequency bidirectional pulses according to the present invention will be illustrated in detail below with reference to the accompanying drawings.

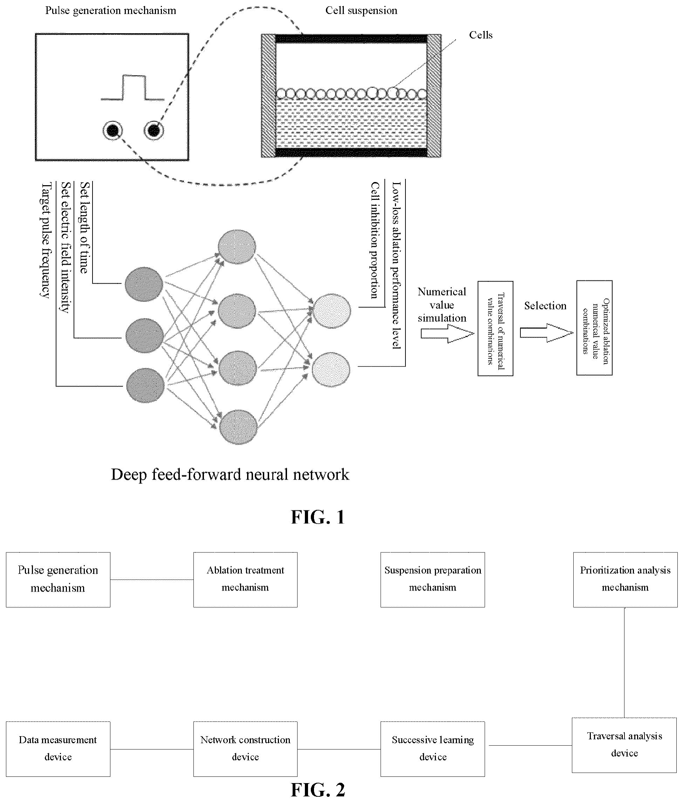

As shown in FIG. 1, it provides the specific inventive conception of an apparatus and a method for testing an electroporation ablation performance using high-frequency bidirectional pulses according to the present invention.

In a first step, an ablation treatment system of a customized structure is established. The ablation treatment system includes a pulse generation mechanism, a suspension preparation mechanism and an ablation treatment mechanism, and the customized structure is configured for promoting the performance and automation level of electroporation ablation treatment using high-frequency bidirectional pulses.

In a second step, an intelligent predictor for predicting an ablation performance is constructed. The intelligent predictor takes the target pulse frequency, the set electric field intensity and the set length of time as used for performing the ablation treatment with the high-frequency bidirectional pulses, as well as the total number of living cells in the struct, as multiple input data, and takes the cell inhibition proportion achieved by the ablation treatment and the low-loss ablation performance level as two output data. The intelligent predictor is based on the deep feed-forward network and completes a set number of times of network learning. In this way, the prediction reliability of the intelligent predictor is ensured.

In a third step, the respective numerical value combinations of the target pulse frequency, the set electric field intensity and the set length of time are traversed for a struct consisting of a fixed total number of living cells from a set part, to thus run the intelligent predictor, respectively, to obtain each predicted ablation treatment performance. The traversing operation is based on the numerical value simulation mode similar to that of the MATLAB toolbox.

In a fourth step, for the struct consisting of a fixed total number of living cells from a set part, the numerical value combination of the target pulse frequency, set electric field intensity and set length of time, corresponding to the optimal ablation treatment performance from the respective predicted ablation treatment performances, is selected as the optimized ablation numerical value combination for the struct of the customized structure. In this way, the cumbersome and massive ablation test analysis processes are effectively replaced.

The key points of the present invention consist in that: the ablation treatment system of the customized structure optimizes the hardware basis for ablation treatment; the established intelligent predictor provides a reliable simulation model for the effective replacement in the subsequent test analysis processes; and the traversal treatment based on numerical value simulation avoids the complex and lengthy alignment of ablation parameters. In this way, multiple optimized ablation parameters can be achieved for each type of struct at relatively higher speed and with reliable accuracy.

The present invention will be exemplarily illustrated in detail below in combination with respective embodiments of the present invention.

FIG. 2 shows a schematic diagram of an internal structure of an apparatus for testing an electroporation ablation performance using high-frequency bidirectional pulses as shown according to a first embodiment of the present invention. The apparatus includes the following components.

A pulse generation mechanism is included. The pulse generation mechanism includes a bipolar pulse generator and a pulse control unit, and the pulse control unit is connected to the bipolar pulse generator and is configured for controlling a pulse generation mode of the bipolar pulse generator to generate high-frequency bidirectional pulses targeting a cell suspension of a cell assembly of interest.

Exemplarily, the specific pulse generation mode of the bipolar pulse generator may be as follows: applying a positive-polarity pulse at first; after a delay time of 2 µs, applying a negative-polarity pulse with the same pulse width; after a latency time of 2 µs, applying a positive-polarity pulse; and repeating this process until the total high level time is 100 µs (50 µs for each of the positive-polarity and negative-polarity pulses), thereby forming a group of pulse train. In the present application, a bipolar pulse mode is used, which consists of positive-polarity and negative-polarity pulses at the same frequency parameter, and these pulses are thus defined as high-frequency bipolar pulses.

Exemplarily, the bipolar pulse generator may be developed by the laboratory itself, an electrode cup used has the width of 4 mm, the feedback signals of voltage and current are collected using a WavePro 760Zi-A oscilloscope, and a PPE-5 kV high-voltage probe and a 6600 Pearson current transformer are provided.

A suspension preparation mechanism is included. The suspension preparation mechanism includes a saline washing device, a digestive treatment device, a centrifuge, a mechanical arm, a timing device, a positioning device, a first preparation container, a second preparation container and a culture formulation device. The suspension preparation mechanism is configured for preparing a cell suspension with a cell population density of 5 × 10⁵ mL⁻¹ by washing a cell assembly of interest two times with a phosphate buffer saline solution with a volume of 1 mL to 2 mL, then digesting the cell assembly of interest for 2 minutes with a pancreatin solution with a mass concentration of 0.5 g/L, then centrifuging with the centrifuge, discarding a supernatant, and adding a 1640 culture medium, and the cell assembly of interest is a struct consisting of a plurality of living cells from the same part.

An ablation treatment mechanism is included. The ablation treatment mechanism is configured for performing ablation treatment on the cell suspension with a set electric field intensity using high-frequency bidirectional pulses at a target pulse frequency, to obtain a treated solution after the ablation treatment has reached a set length of time.

A data measurement device is included. The data measurement device is configured for measuring a total number of remaining living cells, a proportion of necrotic cells, and a proportion of apoptotic cells in the treated solution after the ablation treatment has reached a set length of time, and determining a cell inhibition proportion achieved by the ablation treatment and a low-loss ablation performance level, based on the proportion of necrotic cells and the proportion of apoptotic cells.

A network construction device is included. The network construction device is configured for establishing a deep feed-forward network to perform ablation performance prediction by taking the target pulse frequency, the set electric field intensity and the set length of time as used for performing the ablation treatment using the high-frequency bidirectional pulses, as well as the total number of living cells in the cell assembly of interest, as multiple input data of the deep feed-forward network, and by taking the cell inhibition proportion achieved by the ablation treatment and the low-loss ablation performance level as two output data of the deep feed-forward network.

For example, the deep feed-forward network includes a single input layer, a single output layer and a plurality of hidden layers between the single input layer and the single output layer.

Here, the number of the hidden layers between the single input layer and the single output layer is positively correlated with the total number of living cells in the cell assembly of interest.

A successive learning device is included. The successive learning device is connected to the network construction device and is configured for performing a learning action on the deep feed-forward network by taking the target pulse frequency, the set electric field intensity and the set length of time as used for each ablation treatment, as well as the total number of living cells in the cell assembly of interest, as the multiple input data of the deep feed-forward network, and by taking the cell inhibition proportion achieved by each ablation treatment and the low-loss ablation performance level as two output data of the deep feed-forward network, and outputting the deep feed-forward network that has undergone multiple learning actions up to a preset total number, as an intelligent predictor.

A traversal analysis device is included. The traversal analysis device is connected to the successive learning device and is configured for traversing respective numerical value combinations of the target pulse frequency, the set electric field intensity and the set length of time for the cell assembly of interest consisting of a fixed total number of living cells from a set part, so as to perform a prediction operation on the intelligent predictor of each numerical value combination and thus obtain respective cell inhibition proportions and respective low-loss ablation performance levels corresponding to the respective numerical value combinations. The traversing is based on numerical value simulation treatment.

A prioritization analysis mechanism is included. The prioritization analysis mechanism is connected to the traversal analysis device and is configured for selecting, based on the respective cell inhibition proportions and the respective low-loss ablation performance levels corresponding to the respective numerical value combinations, a numerical value combination with an optimal ablation performance as an optimized ablation numerical value combination for the cell assembly of interest consisting of the fixed total number of living cells from the set part.

Here, determining the cell inhibition proportion achieved by the ablation treatment and the low-loss ablation performance level, based on the proportion of necrotic cells and the proportion of apoptotic cells, includes: summing the proportion of necrotic cells and the proportion of apoptotic cells to obtain the cell inhibition proportion achieved by the ablation treatment, dividing the cell inhibition proportion by the proportion of apoptotic cells to obtain an ablation performance ratio, and determining the low-loss ablation performance level that is in direction proportion to the ablation performance ratio.

Here, selecting, based on the respective cell inhibition proportions and the respective low-loss ablation performance levels corresponding to the respective numerical value combinations, the numerical value combination with the optimal ablation performance as the optimized ablation numerical value combination for the cell assembly of interest consisting of the fixed total number of living cells from the set part includes: selecting a numerical value combination corresponding to a highest cell inhibition proportion as the optimized ablation numerical value combination for the cell assembly of interest consisting of the fixed total number of living cells from the set part.

Here, the network construction device, the successive learning device, the traversal analysis device and the prioritization analysis mechanism may be implemented using different models of SOC chips or CPLD chips, respectively.

Next, the specific structure of the apparatus for testing an electroporation ablation performance using high-frequency bidirectional pulses according to the present invention will be further illustrated in combination with respective embodiments of the present invention.

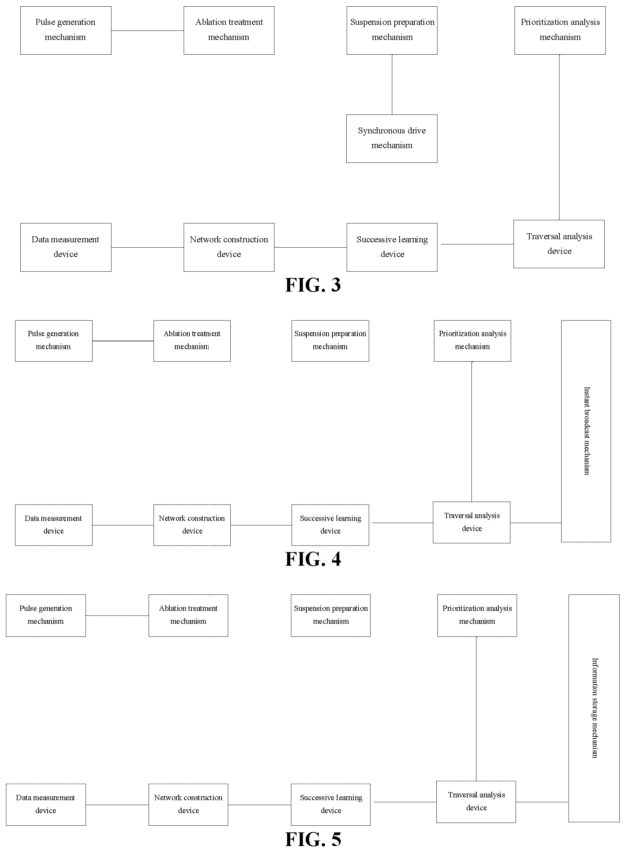

FIG. 3 shows a schematic diagram of an internal structure of an apparatus for testing an electroporation ablation performance using high-frequency bidirectional pulses according to a second embodiment of the present invention. Different from the apparatus for testing an electroporation ablation performance using high-frequency bidirectional pulses as shown according to the first embodiment of the present invention, the apparatus for testing an electroporation ablation performance using high-frequency bidirectional pulses as shown according to the second embodiment of the present invention further includes:
a synchronous drive mechanism, which is connected to the saline washing device, the digestive treatment device, the centrifuge, the mechanical arm, the timing device and the positioning device in the suspension preparation mechanism, respectively.

Here, the synchronous drive mechanism is configured for implementing synchronous action control between every two of the saline washing device, the digestive treatment device, the centrifuge, the mechanical arm, the timing device and the positioning device.

FIG. 4 shows a schematic diagram of an internal structure of an apparatus for testing an electroporation ablation performance using high-frequency bidirectional pulses according to a third embodiment of the present invention. Different from the apparatus for testing an electroporation ablation performance using high-frequency bidirectional pulses as shown according to the first embodiment of the present invention, the apparatus for testing an electroporation ablation performance using high-frequency bidirectional pulses as shown according to the third embodiment of the present invention further includes:
an instant broadcast mechanism, which is connected to the prioritization analysis mechanism and is configured for receiving and broadcasting the optimized ablation numerical value combination for the cell assembly of interest consisting of the fixed total number of living cells from the set part.

Exemplarily, the instant broadcast mechanism includes a voice broadcast unit for broadcasting, in a voice broadcast mode, the optimized ablation numerical value combination for the cell assembly of interest consisting of the fixed total number of living cells from the set part.

Moreover, exemplarily, the instant broadcast mechanism includes an image display unit for broadcasting, in an image display mode, the optimized ablation numerical value combination for the cell assembly of interest consisting of the fixed total number of living cells from the set part.

FIG. 5 shows a schematic diagram of an internal structure of an apparatus for testing an electroporation ablation performance using high-frequency bidirectional pulses according to a fourth embodiment of the present invention. Different from the apparatus for testing an electroporation ablation performance using high-frequency bidirectional pulses as shown according to the first embodiment of the present invention, the apparatus for testing an electroporation ablation performance using high-frequency bidirectional pulses as shown according to the fourth embodiment of the present invention further includes:
an information storage chip, which is connected to the traversal analysis device and is configured for storing respective predictor parameters of the intelligent predictor.

Here, the information storage chip being connected to the traversal analysis device and being configured for storing respective predictor parameters of the intelligent predictor includes: the information storage chip is one of a static storage chip, a TF memory or an MMC memory.

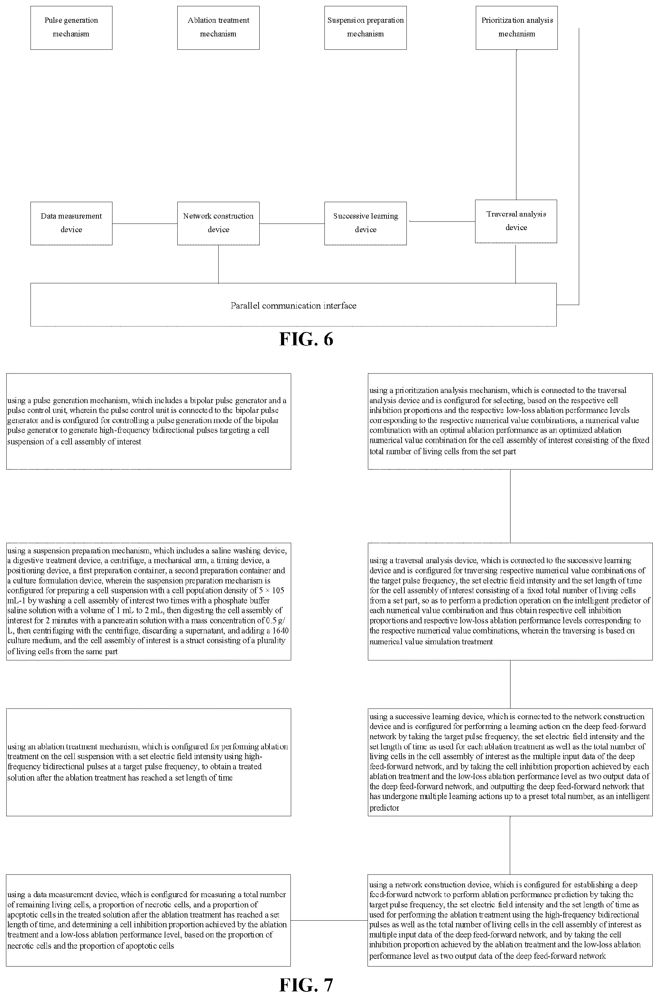

FIG. 6 shows a schematic diagram of an internal structure of an apparatus for testing an electroporation ablation performance using high-frequency bidirectional pulses according to a fifth embodiment of the present invention. Different from the apparatus for testing an electroporation ablation performance using high-frequency bidirectional pulses as shown according to the first embodiment of the present invention, the apparatus for testing an electroporation ablation performance using high-frequency bidirectional pulses as shown according to the fifth embodiment of the present invention further includes:
a parallel communication interface, which is connected to the network construction device, the traversal analysis device and the prioritization analysis mechanism, respectively.

Here, the parallel communication interface is configured for implementing parallel data communication between every two of the network construction device, the traversal analysis device and the prioritization analysis mechanism.

Here, the parallel communication interface being configured for implementing the parallel data communication between every two of the network construction device, the traversal analysis device and the prioritization analysis mechanism includes: the parallel data communication implemented between every two of the network construction device, the traversal analysis device and the prioritization analysis mechanism is 16-bit parallel data communication.

Here, the parallel communication interface being configured for implementing parallel data communication between every two of the network construction device, the traversal analysis device and the prioritization analysis mechanism includes: the parallel communication interface is connected with a serial control interface for controlling the real-time communication mode of the parallel communication interface.

In the apparatus for testing an electroporation ablation performance using high-frequency bidirectional pulses as shown according to any embodiment of the present invention:
establishing the deep feed-forward network to perform ablation performance prediction by taking the target pulse frequency, the set electric field intensity and the set length of time as used for performing the ablation treatment using the high-frequency bidirectional pulses as well as the total number of living cells in the cell assembly of interest as multiple input data of the deep feed-forward network, and by taking the cell inhibition proportion achieved by the ablation treatment and the low-loss ablation performance level as two output data of the deep feed-forward network includes: the target pulse frequency has a reference numerical value of 71.4 kHz, the set electric field intensity has a reference numerical value of 0.2 MV/m, and the set length of time has a reference numerical value of 12 hours; and
controlling the pulse generation mode of the bipolar pulse generator to generate the high-frequency bidirectional pulses targeting the cell suspension of the cell assembly of interest includes: the high-frequency bidirectional pulses have a frequency of more than or equal to 71.4 kHz.

In the apparatus for testing an electroporation ablation performance using high-frequency bidirectional pulses as shown according to any embodiment of the present invention:
preparing the cell suspension with the cell population density of 5 × 10⁵ mL⁻¹ by washing the cell assembly of interest two times with the phosphate buffer saline solution with the volume of 1 mL to 2 mL, then digesting the cell assembly of interest for 2 minutes with the pancreatin solution with the mass concentration of 0.5 g/L, then centrifuging with the centrifuge, discarding the supernatant, and adding the 1640 culture medium includes: the saline washing device is configured for washing the cell assembly of interest two times with the phosphate buffer saline solution with the volume of 1 mL to 2 mL.

Here, preparing the cell suspension with the cell population density of 5 × 10⁵ mL⁻¹ by washing the cell assembly of interest two times with the phosphate buffer saline solution with the volume of 1 mL to 2 mL, then digesting the cell assembly of interest for 2 minutes with the pancreatin solution with the mass concentration of 0.5 g/L, then centrifuging with the centrifuge, discarding the supernatant, and adding the 1640 culture medium further includes: the digestive treatment device is connected to the timing device and is configured for digesting the washed cell assembly of interest for 2 minutes with the pancreatin solution with the mass concentration of 0.5 g/L, and the timing device provides a timing service to the digestive treatment device.

Here, preparing the cell suspension with the cell population density of 5 × 10⁵ mL⁻¹ by washing the cell assembly of interest two times with the phosphate buffer saline solution with the volume of 1 mL to 2 mL, then digesting the cell assembly of interest for 2 minutes with the pancreatin solution with the mass concentration of 0.5 g/L, then centrifuging with the centrifuge, discarding the supernatant, and adding the 1640 culture medium further includes: the centrifuge is configured for centrifuging the solution that has been digested for 2 minutes.

Moreover, in an apparatus for testing an electroporation ablation performance using high-frequency bidirectional pulses as shown according to any embodiment of the present invention:
preparing the cell suspension with the cell population density of 5 × 10⁵ mL⁻¹ by washing the cell assembly of interest two times with the phosphate buffer saline solution with the volume of 1 mL to 2 mL, then digesting the cell assembly of interest for 2 minutes with the pancreatin solution with the mass concentration of 0.5 g/L, then centrifuging with the centrifuge, discarding the supernatant, and adding the 1640 culture medium further includes: the positioning device is connected to the mechanical arm and is configured for providing positioning data for the mechanical arm, and the mechanical arm is configured for clamping the first preparation container containing the solution that has been digested for 2 minutes, clamping the second preparation container containing the solution with the supernatant discarded, or clamping the washed cell assembly of interest.

Here, preparing the cell suspension with the cell population density of 5 × 10⁵ mL⁻¹ by washing the cell assembly of interest two times with the phosphate buffer saline solution with the volume of 1 mL to 2 mL, then digesting the cell assembly of interest for 2 minutes with the pancreatin solution with the mass concentration of 0.5 g/L, then centrifuging with the centrifuge, discarding the supernatant, and adding the 1640 culture medium further includes: the culture formulation device is configured for adding the 1640 culture medium to the second preparation container containing the solution with the supernatant discarded, to formulate the cell suspension with the cell population density of 5 × 10⁵ mL⁻¹.

FIG. 7 shows a schematic diagram of a step process of a method for testing an electroporation ablation performance using high-frequency bidirectional pulses according to a sixth embodiment of the present invention. The method for testing an electroporation ablation performance using high-frequency bidirectional pulses includes steps as follows.

A pulse generation mechanism is used, which includes a bipolar pulse generator and a pulse control unit. The pulse control unit is connected to the bipolar pulse generator and is configured for controlling a pulse generation mode of the bipolar pulse generator to generate high-frequency bidirectional pulses targeting a cell suspension of a cell assembly of interest.

Exemplarily, the specific pulse generation mode of the bipolar pulse generator may be as follows: applying a positive-polarity pulse at first; after a delay time of 2 µs, applying a negative-polarity pulse with the same pulse width; after a latency time of 2 µs, applying a positive-polarity pulse; and repeating this process until the total high level time is 100 µs (50 µs for each of the positive-polarity and negative-polarity pulses), thereby forming a group of pulse train. In the present application, a bipolar pulse mode is used, which consists of positive-polarity and negative-polarity pulses at the same frequency parameter, and these pulses are thus defined as high-frequency bipolar pulses.

Exemplarily, the bipolar pulse generator may be developed by the laboratory itself, an electrode cup used has the width of 4 mm, the feedback signals of voltage and current are collected using a WavePro 760Zi-A oscilloscope, and a PPE-5 kV high-voltage probe and a 6600 Pearson current transformer are provided.

A suspension preparation mechanism is used, which includes a saline washing device, a digestive treatment device, a centrifuge, a mechanical arm, a timing device, a positioning device, a first preparation container, a second preparation container and a culture formulation device. The suspension preparation mechanism is configured for preparing a cell suspension with a cell population density of 5 × 10⁵ mL⁻¹ by washing a cell assembly of interest two times with a phosphate buffer saline solution with a volume of 1 mL to 2 mL, then digesting the cell assembly of interest for 2 minutes with a pancreatin solution with a mass concentration of 0.5 g/L, then centrifuging with the centrifuge, discarding a supernatant, and adding a 1640 culture medium, and the cell assembly of interest is a struct consisting of a plurality of living cells from the same part.

An ablation treatment mechanism is used, which is configured for performing ablation treatment on the cell suspension with a set electric field intensity using high-frequency bidirectional pulses at a target pulse frequency, to obtain a treated solution after the ablation treatment has reached a set length of time.

A data measurement device is used, which is configured for measuring a total number of remaining living cells, a proportion of necrotic cells, and a proportion of apoptotic cells in the treated solution after the ablation treatment has reached a set length of time, and determining a cell inhibition proportion achieved by the ablation treatment and a low-loss ablation performance level, based on the proportion of necrotic cells and the proportion of apoptotic cells.

A network construction device is used, which is configured for establishing a deep feed-forward network to perform ablation performance prediction by taking the target pulse frequency, the set electric field intensity and the set length of time as used for performing the ablation treatment using the high-frequency bidirectional pulses as well as the total number of living cells in the cell assembly of interest as multiple input data of the deep feed-forward network, and by taking the cell inhibition proportion achieved by the ablation treatment and the low-loss ablation performance level as two output data of the deep feed-forward network.

For example, the deep feed-forward network includes a single input layer, a single output layer and a plurality of hidden layers between the single input layer and the single output layer.

Here, the number of the hidden layers between the single input layer and the single output layer is positively correlated with the total number of living cells in the cell assembly of interest.

A successive learning device is used, which is connected to the network construction device and is configured for performing a learning action on the deep feed-forward network by taking the target pulse frequency, the set electric field intensity and the set length of time as used for each ablation treatment as well as the total number of living cells in the cell assembly of interest as the multiple input data of the deep feed-forward network, and by taking the cell inhibition proportion achieved by each ablation treatment and the low-loss ablation performance level as two output data of the deep feed-forward network, and outputting the deep feed-forward network that has undergone multiple learning actions up to a preset total number, as an intelligent predictor.

A traversal analysis device is used, which is connected to the successive learning device and is configured for traversing respective numerical value combinations of the target pulse frequency, the set electric field intensity and the set length of time for the cell assembly of interest consisting of a fixed total number of living cells from a set part, so as to perform a prediction operation on the intelligent predictor of each numerical value combination and thus obtain respective cell inhibition proportions and respective low-loss ablation performance levels corresponding to the respective numerical value combinations. The traversing is based on numerical value simulation treatment.

A prioritization analysis mechanism is used, which is connected to the traversal analysis device and is configured for selecting, based on the respective cell inhibition proportions and the respective low-loss ablation performance levels corresponding to the respective numerical value combinations, a numerical value combination with an optimal ablation performance as an optimized ablation numerical value combination for the cell assembly of interest consisting of the fixed total number of living cells from the set part.

Here, determining the cell inhibition proportion achieved by the ablation treatment and the low-loss ablation performance level, based on the proportion of necrotic cells and the proportion of apoptotic cells, includes: summing the proportion of necrotic cells and the proportion of apoptotic cells to obtain the cell inhibition proportion achieved by the ablation treatment, dividing the cell inhibition proportion by the proportion of apoptotic cells to obtain an ablation performance ratio, and determining the low-loss ablation performance level that is in direction proportion to the ablation performance ratio.

Here, selecting, based on the respective cell inhibition proportions and the respective low-loss ablation performance levels corresponding to the respective numerical value combinations, the numerical value combination with the optimal ablation performance as the optimized ablation numerical value combination for the cell assembly of interest consisting of the fixed total number of living cells from the set part includes: selecting a numerical value combination corresponding to a highest cell inhibition proportion as the optimized ablation numerical value combination for the cell assembly of interest consisting of the fixed total number of living cells from the set part.

Here, the network construction device, the successive learning device, the traversal analysis device and the prioritization analysis mechanism may be implemented using different models of SOC chips or CPLD chips, respectively.

In addition, the present invention may further make reference to the following technical contents to highlight the notable technical advances of the present invention as follows.

Selecting, based on the respective cell inhibition proportions and the respective low-loss ablation performance levels corresponding to the respective numerical value combinations, the numerical value combination with the optimal ablation performance as the optimized ablation numerical value combination for the cell assembly of interest consisting of the fixed total number of living cells from the set part further includes: in the presence of more than two numerical value combinations corresponding to the highest cell inhibition proportions, the numerical value combination in the more than two numerical value combinations corresponding to the highest low-loss ablation performance level is taken as the optimized ablation numerical value combination for the cell assembly of interest consisting of the fixed total number of living cells from the set part.

Here, traversing respective numerical value combinations of the target pulse frequency, the set electric field intensity and the set length of time for the cell assembly of interest consisting of a fixed total number of living cells from a set part, so as to perform a prediction operation on the intelligent predictor of each numerical value combination and thus obtain respective cell inhibition proportions and respective low-loss ablation performance levels corresponding to the respective numerical value combinations, includes: in the prediction operation of the intelligent predictor for each numerical value combination, the intelligent predictor is operated by taking the target pulse frequency, the set electric field intensity, the set length of time and the fixed total count as multiple input data of the intelligent predictor, to obtain the cell inhibition proportion and low-loss ablation performance level outputted by the intelligent predictor, as the prediction results of the prediction operation of the intelligent predictor.

It should be noted that relational terms such as "first" and "second" herein are only intended to distinguish one entity or operation from another entity or operation, and do not necessarily require or imply any such actual relationship or order between these entities or operations. Moreover, the terms "comprise", "include" or any other variants thereof are intended to cover non-exclusive inclusion, such that a process, a method, an article, or a device that includes a series of elements not only includes those elements, but also includes other elements that are not listed explicitly, or also includes elements inherent to this process, method, article or device. In the case of no more limitations, an element defined by a statement "includes a/an..." does not exclude the existence of other same elements in the process, method, article, or device including the element.

The embodiments in the present invention are described in a correlated manner, a mutual reference can be made to the similar or same parts between the embodiments, and each embodiment focuses on the difference of one embodiment from other embodiments. In particular, the embodiments of apparatus/electronic device/computer-readable storage medium/computer program product are described in a simpler way since their embodiments are essentially similar to method embodiments. A reference may be made to the illustration of the method embodiment section for points involved.

Described above are only preferred embodiments of the present invention, and are not intended to limit the protection scope of the present invention. Within the spirit and principles of the present invention, any modifications, equivalent substitutions, improvements and the like are construed as falling within the protection scope of the present invention.

## Claims

1. An apparatus for testing an electroporation ablation performance using high-frequency bidirectional pulses, comprising:
a pulse generation mechanism, which comprises a bipolar pulse generator and a pulse control unit, wherein the pulse control unit is connected to the bipolar pulse generator and is configured for controlling a pulse generation mode of the bipolar pulse generator to generate high-frequency bidirectional pulses targeting a cell suspension of a cell assembly of interest;
a suspension preparation mechanism, which comprises a saline washing device, a digestive treatment device, a centrifuge, a mechanical arm, a timing device, a positioning device, a first preparation container, a second preparation container and a culture formulation device, wherein the suspension preparation mechanism is configured for preparing a cell suspension with a cell population density of 5 × 10⁵ mL⁻¹ by washing a cell assembly of interest two times with a phosphate buffer saline solution with a volume of 1 mL to 2 mL, then digesting the cell assembly of interest for 2 minutes with a pancreatin solution with a mass concentration of 0.5 g/L, then centrifuging with the centrifuge, discarding a supernatant, and adding a 1640 culture medium, and the cell assembly of interest is a struct consisting of a plurality of living cells from the same part;
an ablation treatment mechanism, which is configured for performing ablation treatment on the cell suspension with a set electric field intensity using high-frequency bidirectional pulses at a target pulse frequency, to obtain a treated solution after the ablation treatment has reached a set length of time;
a data measurement device, which is configured for measuring a total number of remaining living cells, a proportion of necrotic cells, and a proportion of apoptotic cells in the treated solution after the ablation treatment has reached a set length of time, and determining a cell inhibition proportion achieved by the ablation treatment and a low-loss ablation performance level, based on the proportion of necrotic cells and the proportion of apoptotic cells;
a network construction device, which is configured for establishing a deep feed-forward network to perform ablation performance prediction by taking the target pulse frequency, the set electric field intensity and the set length of time as used for performing the ablation treatment using the high-frequency bidirectional pulses as well as the total number of living cells in the cell assembly of interest as multiple input data of the deep feed-forward network, and by taking the cell inhibition proportion achieved by the ablation treatment and the low-loss ablation performance level as two output data of the deep feed-forward network;
a successive learning device, which is connected to the network construction device and is configured for performing a learning action on the deep feed-forward network by taking the target pulse frequency, the set electric field intensity and the set length of time as used for each ablation treatment as well as the total number of living cells in the cell assembly of interest as the multiple input data of the deep feed-forward network, and by taking the cell inhibition proportion achieved by each ablation treatment and the low-loss ablation performance level as two output data of the deep feed-forward network, and outputting the deep feed-forward network that has undergone multiple learning actions up to a preset total number, as an intelligent predictor;
a traversal analysis device, which is connected to the successive learning device and is configured for traversing respective numerical value combinations of the target pulse frequency, the set electric field intensity and the set length of time for the cell assembly of interest consisting of a fixed total number of living cells from a set part, so as to perform a prediction operation on the intelligent predictor of each numerical value combination and thus obtain respective cell inhibition proportions and respective low-loss ablation performance levels corresponding to the respective numerical value combinations, wherein the traversing is based on numerical value simulation treatment; and
a prioritization analysis mechanism, which is connected to the traversal analysis device and is configured for selecting, based on the respective cell inhibition proportions and the respective low-loss ablation performance levels corresponding to the respective numerical value combinations, a numerical value combination with an optimal ablation performance as an optimized ablation numerical value combination for the cell assembly of interest consisting of the fixed total number of living cells from the set part.

2. The apparatus for testing an electroporation ablation performance using high-frequency bidirectional pulses according to claim 1, wherein
determining the cell inhibition proportion achieved by the ablation treatment and the low-loss ablation performance level, based on the proportion of necrotic cells and the proportion of apoptotic cells, comprises: summing the proportion of necrotic cells and the proportion of apoptotic cells to obtain the cell inhibition proportion achieved by the ablation treatment, dividing the cell inhibition proportion by the proportion of apoptotic cells to obtain an ablation performance ratio, and determining the low-loss ablation performance level that is in direction proportion to the ablation performance ratio; and
wherein selecting, based on the respective cell inhibition proportions and the respective low-loss ablation performance levels corresponding to the respective numerical value combinations, the numerical value combination with the optimal ablation performance as the optimized ablation numerical value combination for the cell assembly of interest consisting of the fixed total number of living cells from the set part comprises: selecting a numerical value combination corresponding to a highest cell inhibition proportion as the optimized ablation numerical value combination for the cell assembly of interest consisting of the fixed total number of living cells from the set part.

3. The apparatus for testing an electroporation ablation performance using high-frequency bidirectional pulses according to claim 2, further comprising:
a synchronous drive mechanism, which is connected to the saline washing device, the digestive treatment device, the centrifuge, the mechanical arm, the timing device and the positioning device in the suspension preparation mechanism, respectively, and is configured for implementing synchronous action control between every two of the saline washing device, the digestive treatment device, the centrifuge, the mechanical arm, the timing device and the positioning device.

4. The apparatus for testing an electroporation ablation performance using high-frequency bidirectional pulses according to claim 2, further comprising:
an instant broadcast mechanism, which is connected to the prioritization analysis mechanism and is configured for receiving and broadcasting the optimized ablation numerical value combination for the cell assembly of interest consisting of the fixed total number of living cells from the set part.

5. The apparatus for testing an electroporation ablation performance using high-frequency bidirectional pulses according to claim 2, further comprising:
an information storage chip, which is connected to the traversal analysis device and is configured for storing respective predictor parameters of the intelligent predictor.

6. The apparatus for testing an electroporation ablation performance using high-frequency bidirectional pulses according to claim 2, further comprising:
a parallel communication interface, which is connected to the network construction device, the traversal analysis device and the prioritization analysis mechanism, respectively,
wherein the parallel communication interface is configured for implementing parallel data communication between every two of the network construction device, the traversal analysis device and the prioritization analysis mechanism; and
wherein the parallel communication interface being configured for implementing the parallel data communication between every two of the network construction device, the traversal analysis device and the prioritization analysis mechanism comprises: the parallel data communication implemented between every two of the network construction device, the traversal analysis device and the prioritization analysis mechanism is 16-bit parallel data communication.

7. The apparatus for testing an electroporation ablation performance using high-frequency bidirectional pulses according to any one of claims 2 to 6, wherein
establishing the deep feed-forward network to perform ablation performance prediction by taking the target pulse frequency, the set electric field intensity and the set length of time as used for performing the ablation treatment using the high-frequency bidirectional pulses as well as the total number of living cells in the cell assembly of interest as multiple input data of the deep feed-forward network, and by taking the cell inhibition proportion achieved by the ablation treatment and the low-loss ablation performance level as two output data of the deep feed-forward network comprises: the target pulse frequency has a reference numerical value of 71.4 kHz, the set electric field intensity has a reference numerical value of 0.2 MV/m, and the set length of time has a reference numerical value of 12 hours; and
wherein controlling the pulse generation mode of the bipolar pulse generator to generate the high-frequency bidirectional pulses targeting the cell suspension of the cell assembly of interest comprises: the high-frequency bidirectional pulses have a frequency of more than or equal to 71.4 kHz.

8. The apparatus for testing an electroporation ablation performance using high-frequency bidirectional pulses according to any one of claims 2 to 6, wherein
preparing the cell suspension with the cell population density of 5 × 10⁵ mL⁻¹ by washing the cell assembly of interest two times with the phosphate buffer saline solution with the volume of 1 mL to 2 mL, then digesting the cell assembly of interest for 2 minutes with the pancreatin solution with the mass concentration of 0.5 g/L, then centrifuging with the centrifuge, discarding the supernatant, and adding the 1640 culture medium comprises: the saline washing device is configured for washing the cell assembly of interest two times with the phosphate buffer saline solution with the volume of 1 mL to 2 mL;
wherein preparing the cell suspension with the cell population density of 5 × 10⁵ mL⁻¹ by washing the cell assembly of interest two times with the phosphate buffer saline solution with the volume of 1 mL to 2 mL, then digesting the cell assembly of interest for 2 minutes with the pancreatin solution with the mass concentration of 0.5 g/L, then centrifuging with the centrifuge, discarding the supernatant, and adding the 1640 culture medium further comprises: the digestive treatment device is connected to the timing device and is configured for digesting the washed cell assembly of interest for 2 minutes with the pancreatin solution with the mass concentration of 0.5 g/L, and the timing device provides a timing service to the digestive treatment device; and
wherein preparing the cell suspension with the cell population density of 5 × 10⁵ mL⁻¹ by washing the cell assembly of interest two times with the phosphate buffer saline solution with the volume of 1 mL to 2 mL, then digesting the cell assembly of interest for 2 minutes with the pancreatin solution with the mass concentration of 0.5 g/L, then centrifuging with the centrifuge, discarding the supernatant, and adding the 1640 culture medium further comprises: the centrifuge is configured for centrifuging the solution that has been digested for 2 minutes.

9. The apparatus for testing an electroporation ablation performance using high-frequency bidirectional pulses according to claim 8, wherein
preparing the cell suspension with the cell population density of 5 × 10⁵ mL⁻¹ by washing the cell assembly of interest two times with the phosphate buffer saline solution with the volume of 1 mL to 2 mL, then digesting the cell assembly of interest for 2 minutes with the pancreatin solution with the mass concentration of 0.5 g/L, then centrifuging with the centrifuge, discarding the supernatant, and adding the 1640 culture medium further comprises: the positioning device is connected to the mechanical arm and is configured for providing positioning data for the mechanical arm, and the mechanical arm is configured for clamping the first preparation container containing the solution that has been digested for 2 minutes, clamping the second preparation container containing the solution with the supernatant discarded, or clamping the washed cell assembly of interest; and
wherein preparing the cell suspension with the cell population density of 5 × 10⁵ mL⁻¹ by washing the cell assembly of interest two times with the phosphate buffer saline solution with the volume of 1 mL to 2 mL, then digesting the cell assembly of interest for 2 minutes with the pancreatin solution with the mass concentration of 0.5 g/L, then centrifuging with the centrifuge, discarding the supernatant, and adding the 1640 culture medium further comprises: the culture formulation device is configured for adding the 1640 culture medium to the second preparation container containing the solution with the supernatant discarded, to formulate the cell suspension with the cell population density of 5 × 10⁵ mL⁻¹.

10. A method for testing an electroporation ablation performance using high-frequency bidirectional pulses, comprising:
using a pulse generation mechanism, which comprises a bipolar pulse generator and a pulse control unit, wherein the pulse control unit is connected to the bipolar pulse generator and is configured for controlling a pulse generation mode of the bipolar pulse generator to generate high-frequency bidirectional pulses targeting a cell suspension of a cell assembly of interest;
using a suspension preparation mechanism, which comprises a saline washing device, a digestive treatment device, a centrifuge, a mechanical arm, a timing device, a positioning device, a first preparation container, a second preparation container and a culture formulation device, wherein the suspension preparation mechanism is configured for preparing a cell suspension with a cell population density of 5 × 10⁵ mL⁻¹ by washing a cell assembly of interest two times with a phosphate buffer saline solution with a volume of 1 mL to 2 mL, then digesting the cell assembly of interest for 2 minutes with a pancreatin solution with a mass concentration of 0.5 g/L, then centrifuging with the centrifuge, discarding a supernatant, and adding a 1640 culture medium, and the cell assembly of interest is a struct consisting of a plurality of living cells from the same part;
using an ablation treatment mechanism, which is configured for performing ablation treatment on the cell suspension with a set electric field intensity using high-frequency bidirectional pulses at a target pulse frequency, to obtain a treated solution after the ablation treatment has reached a set length of time;
using a data measurement device, which is configured for measuring a total number of remaining living cells, a proportion of necrotic cells, and a proportion of apoptotic cells in the treated solution after the ablation treatment has reached a set length of time, and determining a cell inhibition proportion achieved by the ablation treatment and a low-loss ablation performance level, based on the proportion of necrotic cells and the proportion of apoptotic cells;
using a network construction device, which is configured for establishing a deep feed-forward network to perform ablation performance prediction by taking the target pulse frequency, the set electric field intensity and the set length of time as used for performing the ablation treatment using the high-frequency bidirectional pulses as well as the total number of living cells in the cell assembly of interest as multiple input data of the deep feed-forward network, and by taking the cell inhibition proportion achieved by the ablation treatment and the low-loss ablation performance level as two output data of the deep feed-forward network;
using a successive learning device, which is connected to the network construction device and is configured for performing a learning action on the deep feed-forward network by taking the target pulse frequency, the set electric field intensity and the set length of time as used for each ablation treatment as well as the total number of living cells in the cell assembly of interest as the multiple input data of the deep feed-forward network, and by taking the cell inhibition proportion achieved by each ablation treatment and the low-loss ablation performance level as two output data of the deep feed-forward network, and outputting the deep feed-forward network that has undergone multiple learning actions up to a preset total number, as an intelligent predictor;
using a traversal analysis device, which is connected to the successive learning device and is configured for traversing respective numerical value combinations of the target pulse frequency, the set electric field intensity and the set length of time for the cell assembly of interest consisting of a fixed total number of living cells from a set part, so as to perform a prediction operation on the intelligent predictor of each numerical value combination and thus obtain respective cell inhibition proportions and respective low-loss ablation performance levels corresponding to the respective numerical value combinations, wherein the traversing is based on numerical value simulation treatment; and
using a prioritization analysis mechanism, which is connected to the traversal analysis device and is configured for selecting, based on the respective cell inhibition proportions and the respective low-loss ablation performance levels corresponding to the respective numerical value combinations, a numerical value combination with an optimal ablation performance as an optimized ablation numerical value combination for the cell assembly of interest consisting of the fixed total number of living cells from the set part.
